# EUROPEAN PATENT SPECIFICATION

(11) **EP 0 637 257 B1**
(45) Date of publication and mention of the grant of the patent: **29.11.2000**
(21) Application number: 93910733.0
(22) Date of filing: 21.04.1993
(51) Int. Cl.: A61M 16/00, A61M 16/04, A61M 16/20

(54) **RESPIRATORY SUPPORT SYSTEM**
ATMUNGSUNTERSTÜTZUNGSSYSTEM
SYSTEME D'ASSISTANCE RESPIRATOIRE

(30) Priority: 24.04.1992 US 873470; 19.10.1992 US 962755; 19.10.1992 US 962756; 19.10.1992 US 962757
(43) Date of publication of application: 08.02.1995
(73) Proprietor: SHERWOOD MEDICAL COMPANY, St. Louis, MO 63103-1642 (US)
(72) Inventor: SCHNEIDER, James, St. Louis, MO 63146 (US); KEE, Kok-Hiong, St. Louis, MO 63021 (US); KOLLER, NEAL, G., St. Louis, MO 63130 (US); BRUNO, Robert, H., Avon, CT 06001 (US)
(74) Representative: Chettle, Adrian John
(86) International application number: US9303802
(87) International publication number: WO9321981

(56) References cited:
- EP-A- 0 414 997
- DE-A- 1 912 287
- DE-A- 3 629 857
- FR-A- 2 528 707
- GB-A- 2 207 736
- US-A- 3 526 249
- US-A- 3 991 762
- US-A- 4 356 823
- US-A- 4 850 350
- US-A- 5 083 561
- US-A- 5 088 486

## Description

### TECHNICAL FIELD

### 1. Field of the Invention

This invention relates generally to a respiratory support system according to the preamble of claim 1.

### 2. Prior Art

Respiratory support systems used for the ventilation of critically ill patients are now commonly used in medical facilities. Typically, a prior art respiratory support system includes a tracheal tube positioned either directly, or through the nose or mouth, into the trachea of a patient, a manifold connected to the tracheal tube at one port position thereof, and a source of breathable gas connected at a second port thereof. The purpose of the respiratory support system is to assist the patient in maintaining adequate blood oxygenation levels without overtaxing the patient's heart and lungs.

While a patient is attached to the respiratory support system, it is periodically necessary to aspirate fluid from the patient's trachea or lungs. In the past, in order to accomplish aspiration, it has been necessary to disassemble part of the respiratory support system, either by removing the ventilator manifold therefrom or by opening a port of the manifold and inserting a small diameter suction tube down the tracheal tube and into the patient's trachea and lungs. The fluid was then suctioned from the patient and the suction catheter was removed and the respiratory support system reassembled. However, due to the interruption of respiratory support during this procedure, a patient's blood oxygen often dropped to an unacceptably low level, even when other previously known breathing assistance efforts were simultaneously provided.

One solution to the above problem, which is generally exemplary of the prior art, is shown in U.S. Patent No. 5,073,164 to Hollister et al., which includes a ventilator manifold having an access port therethrough which is adapted to receive a connector of the suction catheter device. The suction catheter device positions a catheter within the ventilator manifold without substantial manifold pressure loss. The suction catheter device includes an envelope which is positioned around the catheter portion thereof in order to prevent contamination of catheter surfaces intended to be inserted into the patient's trachea and lungs.

Although this type of ventilator manifold and suction catheter device connection allows continuous respiratory support of the patient during suctioning of fluid from the patient's trachea and lungs, it nevertheless has several drawbacks associated with its use. For example, removal of the suction catheter device from the manifold, such as for the purpose of replacing the auction catheter device, or for attaching another accessory to the manifold (e.g., a manual resuscitation bag or a metered dose inhaler) cannot be accomplished without loss of internal manifold pressure and thereby a compromise of the integrity of the respiratory system. Further, separation of the Hollister et al. suction catheter device from their suction control valve cannot be accomplished without opening the manifold to atmospheric pressure through the catheter. Therefore, replacement of either the suction catheter device or the suction control valve is not possible without loss of internal manifold pressure. Instead, respiratory support of the patient is compromised whenever the suction catheter device or the suction control valve is removed from the system for any reason. Since the suction catheter device tends to become contaminated relatively quickly with respect to the suction control valve and the ventilator manifold, it must be changed out of the system and replaced on a relatively frequent basis. However, because of the problems caused by loss of respiratory support during replacement, the ventilator manifold and/or the suction control valve are often prematurely discarded along with the suction catheter device in order to limit replacement time and the number of replacement procedures required.

U.S. Patent No. 4,351,328 to Bodai attempts to solve one of the above problems by forming an opening in the ventilator manifold which is blocked by a pre-punctured resilient seal through which a catheter can be passed without substantially affecting the integrity of the system, i.e., without substantial gas exchange or pressure loss between the interior of the manifold and the atmosphere. The Bodai device, although allowing entry and removal of a suction catheter through the ventilator manifold during continuous respiratory support of a patient, nevertheless fails to completely resolve the existing problems in the prior art. Specifically, the pre-punctured resilient material in Bodai's manifold opening allows only for the insertion of a catheter therethrough, and fails to accommodate a suction catheter device which includes a collapsible envelope which surrounds and seals the catheter against exterior surface contamination. Further, there is no design consideration for the attachment of other accessory devices to the manifold, such as a manual resuscitation bag or a metered dose inhaler, which are often necessary for use in the care of a patient.

Also, the system described by Bodai tends to cause mucus and other fluids from the patient's lungs and trachea to collect in the manifold as the catheter is pulled past the pre-punctured resilient seal when being withdrawn. Because of this contamination problem, it is often necessary to replace the manifold on a more frequent basis than would otherwise be necessary, which necessitates a pressure breach in the support system.

There therefore exists a need in the art for a respiratory support system which includes a ventilator manifold which allows simple attachment and detachment of a suction catheter device therefrom during continuous patient respiratory support, without substantial pressure loss from the manifold and without substantial collection of body fluids in the manifold. There also exists a need in the art for a suction catheter device and a suction control valve which can be disassembled and reassembled, individually or collectively, from the respiratory support system during use thereof, and reassembled or replaced thereafter, without causing interior pressure loss from the ventilator manifold.

FR-A-2528707 discloses a system according to he preamble of claim 1.

### DISCLOSURE OF INVENTION

The invention is defined by the characterising features of claim 1. Preferred features are disclosed in the subclaims.

The invention provides a suction catheter device which is designed to be capable of interchangeably engaging and disengaging a normally closed valve of a manifold port of a respiratory support system at one end thereof, and a suction control valve at the other end thereof, without comprising internal manifold pressure integrity.

The access port and adaptor may include a detent and stop-type locking arrangement for locking the adaptor within the port against inadvertent withdrawal thereof during use, and for orienting the adaptor in a single unique position relative to the access port to align the side opening through the side of the access port with an opening through the side of the adaptor which allows cleaning and/or lavage fluid to be injected into the interior of the adaptor and/or the interior of the manifold if desired.

The catheter device may include a dual-lumen catheter for suction and irrigation purposes, and the valve-end connector therefore can include a one-way saline injection port attached in fluid flow connection with the fluid injection lumen of the dual-lumen catheter.

### BRIEF DESCRIPTION OF DRAWINGS

Figure 1 shows a suction control valve and a manifold of a respiratory support system attached for use to a suction catheter device formed in accordance with the principles of the present invention;
Figure 2 is a plan view of a portion of the suction catheter device which includes the manifold-end connector thereof formed in accordance with the principles of the present invention;
Figure 3 is a cross-sectional view of the manifold-end connector of the suction catheter device shown in Figure 2;
Figure 4 is a side view of the ventilator manifold;
Figure 5 is a cross-sectional view of the ventilator manifold shown in Figure 4;
Figure 6 is a cross-sectional view of the ventilator manifold with the manifold end connector of the suction catheter device attached to the access port thereof;
Figure 7 is a cross-sectional view of the valve-end connector of the suction catheter device formed in accordance with the principles of the present invention;
Figure 8 is a cross-sectional view of the housing portion of the valve-end connector;
Figure 9 is a cross-sectional view of the insert portion of the valve-end connector;
Figure 10 is a plan view of an alternative embodiment of the suction catheter device formed in accordance with the principles of the present invention;
Figure 11 is a cross-sectional view of the valve-end connector of the alternative embodiment of the suction catheter device shown in Figure 10;
Figure 12 is a perspective view of a suction control valve formed in accordance with the principles of the present invention;
Figure 13 is a cross-sectional view of the fluid flow valving device of Figure 12;
Figure 14 is a cross-sectional view of the valve housing of the fluid flow valving device of the present invention;
Figure 15 is a cross-sectional view of the valve body of the fluid flow valving device of the present invention;
Figure 16 is a top view of the rotatable core of the fluid flow valving device formed in accordance with the principles of the present invention; and
Figure 17 is a cross-sectional view of the fluid flow valving device as shown in Figure 13, showing the actuator and core rotated to the locked position.

### MODE FOR CARRYING OUT THE INVENTION

As shown in the exemplary drawings for the purposes of illustration, an embodiment of a manifold, suction catheter, and suction control valve of a respiratory support system made in accordance with the principles of the present invention, referred to generally by the reference numeral 10, is provided for interchangeable use of either the suction catheter or the suction control valve without interruption of respiratory support of the patient.

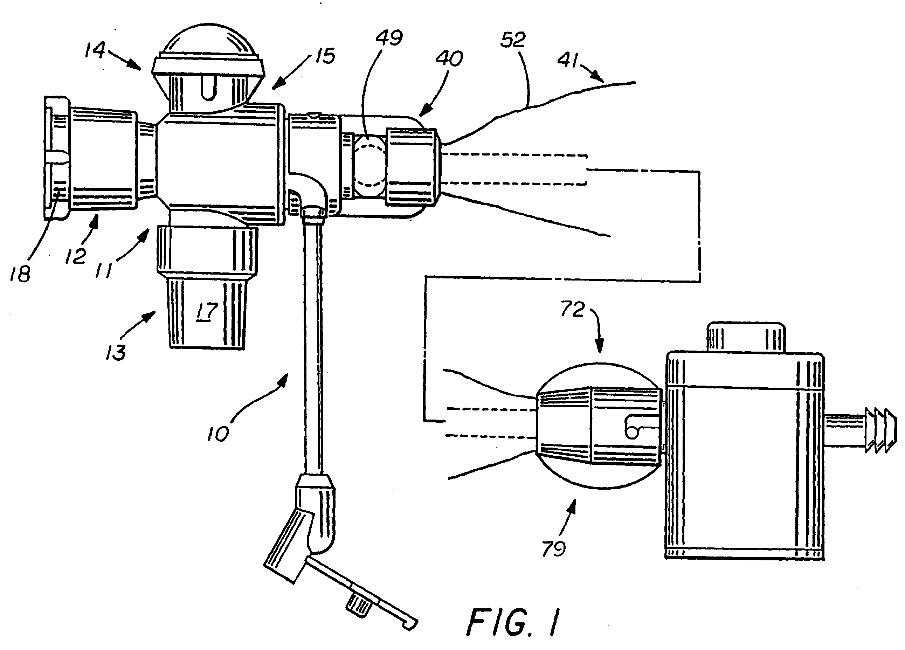

More specifically, as shown in Figure 1, the ventilator manifold 11 includes a plurality of access ports which facilitate-its connection to a ventilator circuit which is in use by the patient. The manifold 11 is attached to a patient for fluid flow communication with the patient's lungs by the connection of the patient attachment port 12 thereof to the connector of an endotracheal tube assembly (not shown) which has been previously positioned in the trachea of a patient by any one of several well known procedures.

Ventilator circuit connection port 13 of the manifold 11 is designed for connection to flexible breathing hoses from the ventilator (not shown) in a well-known manner, such as through a "Y" site connector. Port 14 is normally capped and closed against air flow except for instances when nonpressurized ventilation is desired. The ventilator circuit provides a breathable gas mixture to the patient through one hose, and receives expelled air from the patient's lungs through another hose. The ventilator circuit further commonly includes various valves, regulators and the like associated with the hoses in order to effect respiration of the patient. The manifold 11, and hoses attached thereto at the ventilator circuit connection port 12, are generally made of disposable plastic material and are generally intended to be used by only one patient and then discarded.

When attached to the patient, the entire respiratory support system 10 is designed to isolate the patient's lungs from the atmosphere and allow pressurized forced ventilation of a gas mixture of a high oxygen content from the ventilator into the patient's lungs. Commonly ventilators of this type are used to maintain a positive end expiratory pressure (PEEP) within the ventilator manifold 11 and the patient's lungs at all times during exhalation. This technique is commonly used because of the benefit of supplying a minimum concentration of oxygen to the patient at all times for maintaining a proper blood oxygenation level. The PEEP procedure also keeps a large number of lung alveoli of the patient open at all times during respiratory support, thus increasing the effective lung area subject to ventilation.

Prevailing respiratory support techniques including PEEP, have made it very disadvantageous to interrupt respiratory support to the patient by opening the ventilator manifold to the atmosphere and thereby causing a loss of interior manifold pressure. Therefor, the necessary attachment of accessory devices thereto for medical procedures has been difficult to the loss of isolation of the respiratory system from the atmosphere during these procedures, and the immediate loss of effective lung surface area due to collapse of the patient's lung alveoli. Further, when such procedures have been prolonged for any reason, the patient's blood oxygen has often dropped to inadequate levels, and subsequently forced overexertion of the patient's lungs and heart in order to return the blood oxygen level to normal. Also, disassembly and reassembly of the respiratory support system components for procedures with prior art accessory devices has often been a very time consuming procedure for the medical worker involved.

The present invention resolves the problems associated with loss of isolation of the respiratory support system from the atmosphere when accessory devices must be inserted or attached in order to perform necessary medical procedures, or alternatively, when they must be replaced, during respiratory support of a patient.

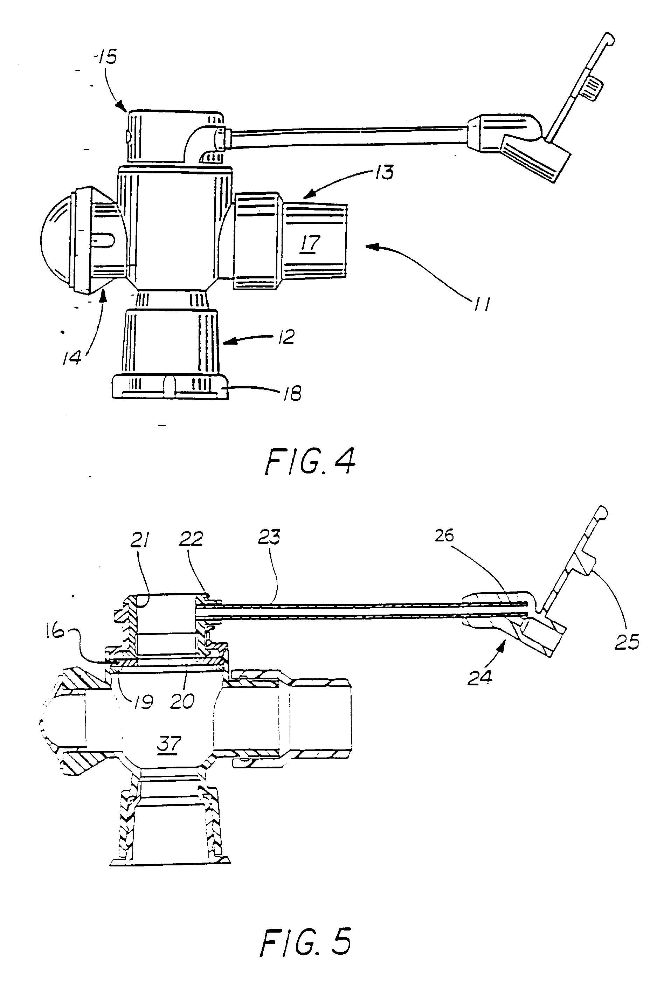

Specifically, the manifold 11 of the present invention includes an access port 15 which is in fluid flow communication with the interior of the manifold 11. The access port 15 includes a normally closed valve 16 (see Figure 5) preferably made of a resilient material such as rubber or silicone which maintains the interior of the manifold 11 isolated from the atmosphere at all times. As explained above, the interior of the manifold 11, although experiencing constant pressure fluctuations, is generally kept at a pressure which is slightly above atmospheric pressure in order to properly administer oxygen according to the PEEP procedure.

The ventilator circuit connection port 13 and the patient attachment port 12 may, if desired, include swivel connectors 17 and 18 respectively thereon in order to allow relative rotation between the manifold 11 and the trachea tube and breathing hoses in order to isolate the trachea tube from the incidental forces causes by the manifold 11 or the breathing hoses attached thereto so as to increase the comfort of the patient.

As best shown in Figures 4 and 5, the access port 15 includes a normally closed valve 16 formed therein which maintains the interior of the manifold 11 isolated from the atmosphere at all times. As explained above, the interior of the manifold 11, although experiencing constant pressure fluctuations, is generally kept at a pressure which is slightly above atmospheric pressure in order to properly administer oxygen according to the PEEP procedure. Therefore, the valve 16 is preferably made of a resilient material to ensure that pressure isolation of the manifold 11 is maintained.

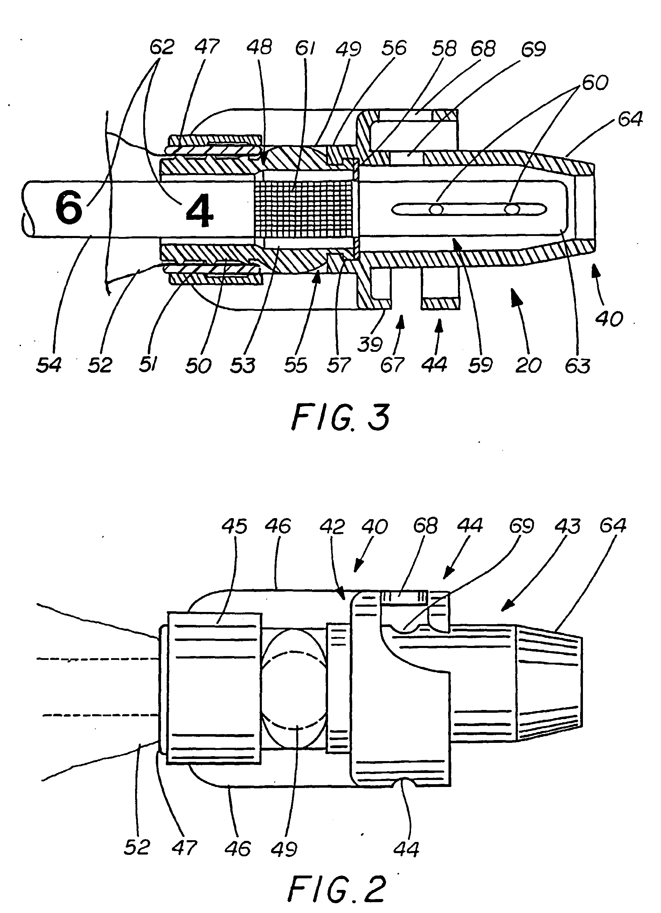

The valve 16 is preferably formed to a circular disk shape and inserted into the manifold 11 between the access port 15 and a support ring 19. The valve 16 is formed with a slit, or a pair of perpendicular slits 20 which are normally closed against fluid flow therethrough, but may be forced opened by the insertion of the manifold end connector 40 therethrough (as shown in Figure 3), of the suction catheter device 41.

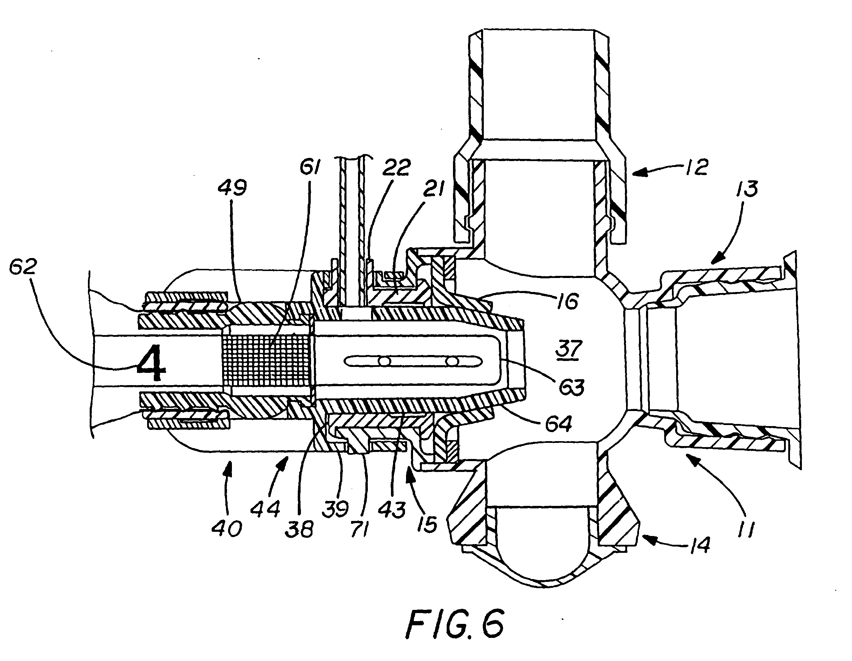

The interior of the access port 15 is lined with a sleeve member 21 which covers the entire interior surface of the access port 15 and abuts in sealing relationship against the normally closed valve 16. The interior diameter of the sleeve member 21 is predetermined to cause a snug fit with the manifold end connector 40 (as best shown in Figure 6) to assist in the prevention of leakage from the manifold 11 when the normally closed valve 16 is forced opened by the manifold end connector 40.

The access port 15 forms a side opening 22 therethrough through which a portion of the sleeve 21 extends to be attached, such as by solvent bonding, to a pigtail fluid injection tube 23 which is intended for use in transporting fluid through the access port side opening 22 into the interior of the access port 15. The opposite end of the pigtail tube 23 includes a luer connector 24 attached thereto with an integrally formed luer connector plug 25. A check valve 26, taking the form of a collapsible sleeve, may be positioned between the luer connector 24 and the pigtail tube 23 if desired, to collapse upon injection of fluid through the luer connector 24 into the pigtail tube 23, but expand to block fluid flow in the opposite direction.

It is preferred that the sleeve member 21 be formed of a relatively flexible material such as plasticized PVC, having good solvent bonding characteristics with the material forming the pigtail tube 23, the pigtail tube 23 preferably is formed of the same material as the sleeve member 21. The access port 15 according to the preferred embodiment of the present invention is preferably formed of clear ABS, which is preferably the same material forming the main body of the manifold 11 in order to ensure good ultrasonic or solvent bonding therebetween.

Referring now to Figures 2 and 3, the manifold-end connector 40 of the suction catheter device 41 is shown. The connector 40 includes a unitary housing 42 which forms an adaptor 43, a locking mechanism 44, a base ring 45, and a plurality of rib members 46. The base ring 45 forms a generally cylindrical opening through which the sleeve attachment ring 47 can be inserted for frictional engagement to hold the sleeve 52 in proper position relative to the connector 40.

A magnifying insert 48 is formed as a generally cylindrical member having a bulbous lens 49 at one end thereof formed of clear plastic and a cylindrical extension 50 formed at the other end thereof. The cylindrical extension 50 is formed to a slightly smaller diameter than the sleeve attachment ring 47 and may include ribbing 51 around the exterior surface thereof to aid in frictional engagement between the extension 50 and the sleeve attachment ring 47. When constructed for use, the sleeve 52 is positioned around the sleeve attachment ring 47 so as to be frictionally engaged between the extension 50 and the interior surface of the sleeve attachment ring 47, and wrapped around at least a portion of the exterior surface of the sleeve attachment ring 47 to also become frictionally engaged with the base ring 45 when the sleeve attachment ring 47 is inserted therein.

The lens 49 of the magnifying insert 48 is preferably formed of a substantially clear plastic which magnifies the portion of the catheter 54 for viewing by a user through the generally cylindrical opening or window 55 formed by the unitary housing 42 between the base ring 45 and the locking mechanism 44. The window 55 extends around the entire circumference of the unitary housing 42 and allows viewing of the lens 49 by a user at any viewing angle except where the window 55 may be slightly covered by a portion of the ribbed members 46 which extend between the base ring 45 and the locking mechanism 44.

The unitary housing 42 also includes an annular locking shoulder 56 which operates in conjunction with an annular locking shoulder 57 on the magnifying insert 48 to secure the magnifying insert 48 in proper position within the unitary housing 42, and to ensure an air-tight seal therebetween and with the sealing ring 58.

The magnifying insert 48 has a generally cylindrical passageway 53 formed therethrough which is of slightly larger diameter than the catheter 54 and which allows uninhibited movement of the catheter 54 therethrough.

If desired, the catheter 54 nay be formed with a tip 59 of softer material than the remainder of the catheter 54 and which may include side openings 60 therein. The catheter 54 may also include a series of markings such as ring marking 61 and/or number markings 62 along the length thereof which will tend to be magnified when located beneath the lens 49. The marking 61 is intended to indicate the completely withdrawn position of the catheter 54 into the adaptor 43.

For example, in operation, the user can withdraw the catheter 54 through the manifold-end connector 40 until the ring marking 61 moves into view within the lens 49. Positioning of the ring marking 61 beneath the lens 49 indicates to the user that the catheter 54 has been withdrawn the entire recommended distance through the connector 40 and cannot be further withdrawn without risking inadvertent passage of the side openings 60 of the catheter 54 past the sealing ring 58, which would effectively allow leakage of air past the sealing ring 58. As is readily evident, an even greater leakage of air would occur if withdrawal of the catheter 54 continued until the distal end 63 thereof passed through the sealing ring 58 or through the connector 40 entirely.

The number markings 62 may be positioned along the catheter 54 so as to indicate to the user a particular predetermined distance which the distal end 63 thereof extends beyond the connector 40. As each number marking 62 appears in the lens 49, the user can recognize the number as corresponding to a particular predetermined distance that the distal end 63 is extending beyond the connector 40. In this manner, when the connector 40 is attached to the manifold 11 of the respiratory support system 10, the user can readily determine how far down a patient's trachea or lungs the catheter 54 has been inserted during an aspiration procedure by noting the particular number marking 62 visible through the lens 49.

The adapter 43 and locking mechanism 44 of the connector 40 operate to attach the connector 40 to the ventilator manifold 11. As best shown in Figure 6, attachment of the connector 40 to the manifold 11 is effected by insertion of the adapter 43 into the access port 15 until the tapered top section 64 thereof engages the valve 16 and forces it toward the interior of the manifold 11. Upon complete insertion of the adapter 43 into the access port 15, the valve 16 is completely open.

In reference to Figures 3 and 6, the locking mechanism 44 of the connector 40 is formed to encircle a portion of the adapter 43 and includes a pair of arcuate slots 67 and 68 which operate together to ensure secure attachment of the connector 40 to the access port 15 of the manifold 11, and also ensure proper relative orientation between the adapter 43 and the access port 15, to cause the injection fluid opening 69 of the adapter 43 to be positioned in alignment with the side opening 22 of the access port 15 when the adapter 43 is properly locked in position therein for use. The arcuate slot 67 is sized to be engageable with the nub 71 which is located directly opposite the side opening 22 on the access port 15. The arcuate slot 68 is larger in width than the arcuate slot 67 and therefore can accommodate the side opening 22 of the access port 15. As is readily evident, the adapter 43 can only be locked in position within the access port 15 in one unique relative orientation in which the injection fluid opening 69 and the side opening 22 are in alignment.

As best shown in Figure 6, attachment of the manifold-end connector 40 to the respiratory manifold 11 is effected by insertion of the adaptor 43 into the access port 15 until the tapered top section 64 engages the valve 16 and forces it toward the interior of the manifold 11. Upon complete insertion of the adaptor 43 into the port 15, the valve 16 is completely open and the elastic sleeve member 21 sealingly engaged with the adaptor 43. Also, the sleeve shoulder 38 of the sleeve member 21 is forced to resiliently deform within the base 39 of the locking mechanism 44. This increases the air tight seal and assists in positively locking the adaptor 11 to the access port 15 by forcing the arcuate slots 67 and 68 against the nub 71 and side opening 22 respectively.

It is intended that during insertion of the adaptor 11 into the access port 15, the sealing relationship formed between the sleeve member 21 and the adaptor 43 commence prior to opening of the valve 16 by the tapered top section 64, in order to ensure isolation of the interior of the manifold 11 from the atmosphere during attachment of the suction catheter device 41. Once completely inserted within the port 15, the tapered top section 64 extends completely through the access port 15 and into the manifold central chamber 37.

As shown in Figure 4, the pigtail tube 23 can be used to inject fluid into the adaptor 43 to clean the suction catheter 54 and the sealing ring 58 of mucal materials which may have accumulated therein due to repeated insertion and withdrawn of the catheter 54 from the patient's lungs during aspiration procedures. The cleaning fluid can then be aspirated through the catheter 54 to remove it from the interior of the adaptor 43 and the manifold 11.

Alternatively, if desired, fluid may be injected through the pigtail tube 23, into the adaptor 43 and through the central chamber 37 of the manifold 11, and through the patient connection port 12 into the trachea and lungs of the patient for purposes of lavage. The suction catheter 54 can then be inserted through the manifold 11 into the patient's trachea and the fluid can be aspirated along with any mucal materials dislodged by the lavage fluid, as will be explained below.

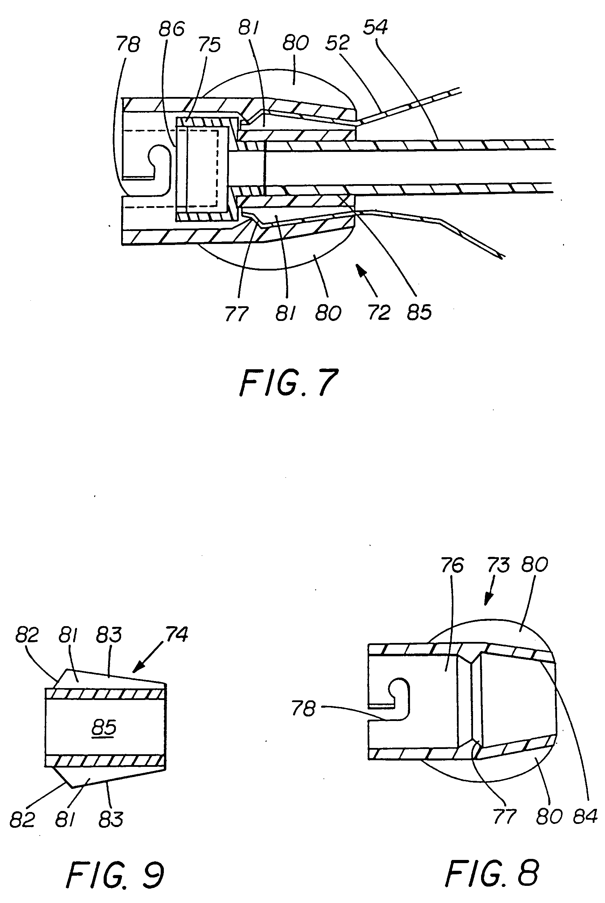

Referring now to Figures 7-9, the valve-end connector 72 of the suction catheter device 41 is shown. The connector 72 includes three main components including a housing 73, a snap-in insert 74, and a slit septum 75. The housing 73 forms a passageway 76 therethrough which has an annular shoulder 77 protruding thereinto at an approximately centrally located position along the passageway 76. The housing 73 also includes a pair of locking slots 78 for attachment of the connector 72 to a suction control valve 79 (shown in dashed lines in Figure 7) and a pair of longitudinally oriented finger grips 80 which facilitate rotation of the connector 72 for attachment to the suction control valve 79 (see Fig. 1).

The snap-in insert 74 is a generally cylindrical tubular member having a plurality of uniformly spaced fins 81 positioned longitudinally therealong in a plurality of uniformly spaced locations. Each fin 81 includes a locking shoulder 82 at one end thereof and a tapered edge 83 along the length thereof. The locking shoulder 82 and tapered edge 83 are sized to match the annular shoulder 77 and the tapered portion 84 of the housing passageway 76 so as to securely hold the catheter sleeve 52 in a friction fit within the housing 73 between the fins 81 and the tapered portion 84 of the passageway 76.

The snap-in insert 74 also forms a tubular channel 85 therein which is sized to accommodate the proximal end of the catheter 54 and the distal end of the slit septum 75 in a permanently attached manner such as by solvent bonding or the like. The slit 86 in the septum may be of any desired shape or configuration such a linear, curvelinear, cross, or the like, which can be easily pushed open by the suction control valve 79 and which will return to its closed position upon its removal.

It should be noted that the connector 72 intentionally forms an open air flow path from the interior of the sleeve 52 snap-in insert 74 between the fins 81 and around the slitted septum 75 past the suction control valve 79 to the atmosphere. This air flow path is intentionally designed to ensure that the sleeve 52 is not sealed against air flow between the interior thereof and the atmosphere. This prevents pressure or vacuum build up within the sleeve 52 during operation of the suction catheter device 41 due to contraction and expansion of the sleeve 52 caused by the movement of the catheter 54 through the respiratory manifold 11.

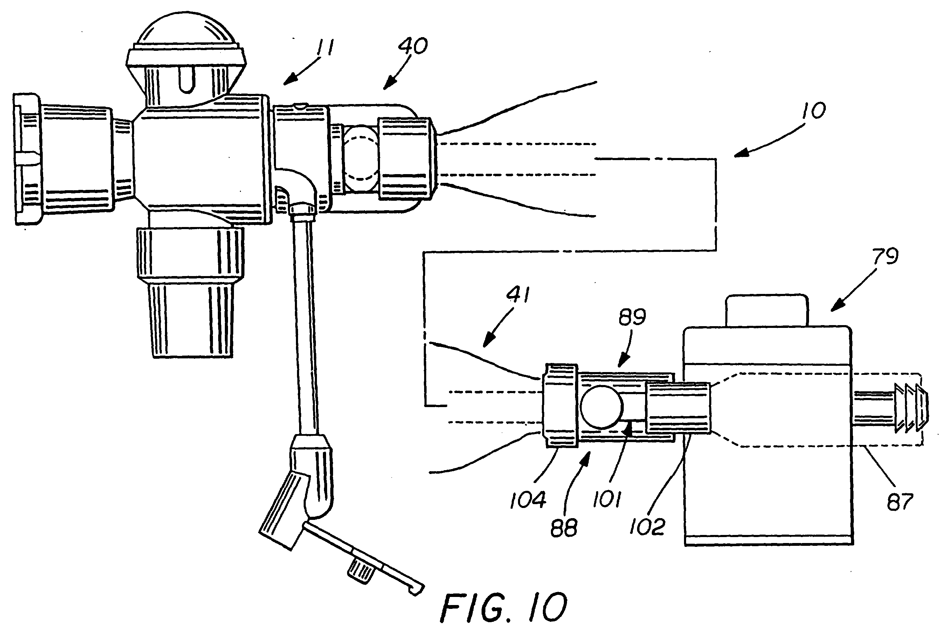

Referring now to Figure 10, an alternative embodiment of the respiratory support system 10 is shown which is substantially identical to the respiratory support system 10 described above, except that the catheter thereof is a dual-lumen catheter and the valve-end connector is modified to allow injection of fluid from a flexible fluid vial 87 through the second lumen.

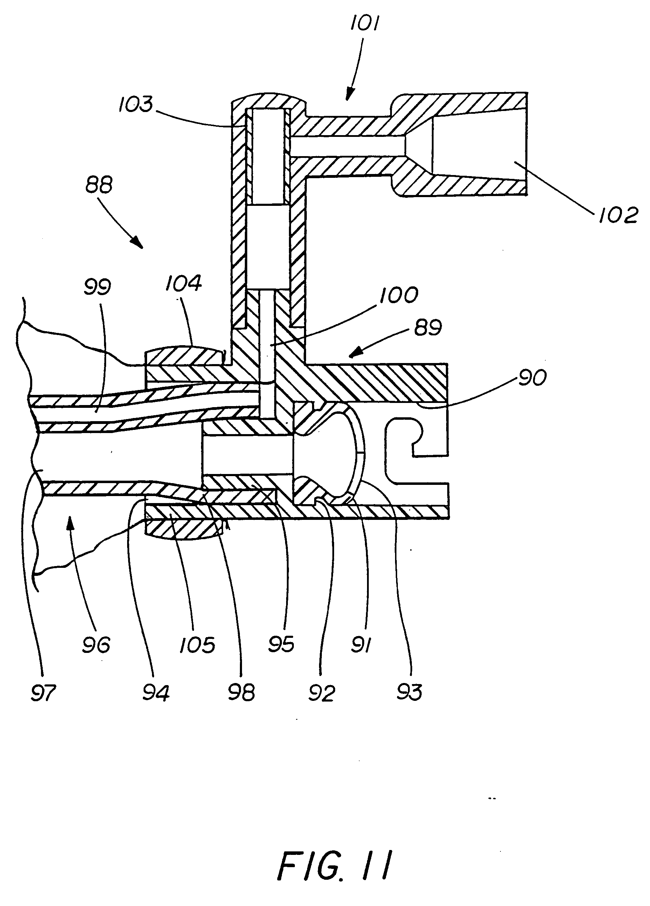

More specifically, as shown in Figure 11, the dual-lumen valve-end connector 88 includes a housing 89 which forms a generally cylindrical opening 90 into which the suction control valve (shown in dashed lines) can be inserted for connection, and in which a slitted septum 91 is affixed such as by annular shoulder 92. The slitted septum 91 includes a normally closed slit 93 therein which is opened by the insertion of the suction control valve in the manner described previously with respect to the single-lumen valve-end connector 72 above.

The housing 89 also includes a second cylindrical opening 94 which has a tubular extension 95 formed therein. The opening 94 is sized to receive the proximal end of the dual-lumen catheter 96, and the tubular extension 95 is designed to be inserted within the larger (suction) lumen 97 of the proximal end 98 of the dual-lumen catheter 96.

As is evident in Figure 11, the proximal end 98 of the dual-lumen catheter 96 is somewhat larger in diameter than the remainder of the catheter 96. This is preferably due to intentional manufacturing of the catheter 96 with an enlarged proximal end 98, and not necessarily due only to stretching of the proximal end 98 about the tubular extension 95. This is a desirable feature of the present invention in that intentional over-sizing of the proximal end 98 of the catheter 96 helps avoid any restricted diameter areas along the large lumen 97, and also helps prevent the attachment of the large lumen 97 over the tubular extension 95 from causing a restriction in the diameter of the smaller (irrigation) lumen 99 at the proximal end 98.

A flow channel 100 extends away from the bottom of the second cylindrical opening 94 and is in fluid flow communication with the small lumen 99 of the catheter 96. The flow channel 100 communicates with an L-shaped tubular member 101 which includes a luer-type connection opening 102 designed to receive the flexible fluid vial 87 (shown in Figure 10). The L-shaped tubular member 101 includes a one-way valve 103 therein preferably formed as a soft tubular sleeve which collapses when pressurized and allows fluid to be injected into the small lumen 99 of the catheter 96 through the flow channel 100, but inhibits fluid flow in the opposite direction.

The catheter sleeve 52, which surrounds the catheter 96, is affixed to the housing 89 by the attachment ring 104, in a friction fit manner, and an air flow path from inside the sleeve 52 through the connector 88 to the atmosphere is formed by the air flow channel 105.

The preferred manner of assembly of the suction catheter device 41 of the present invention is as follows. First, depending on whether a single-lumen or dual-lumen catheter is used, the single-lumen or dual-lumen valve-end connector 72 or 88, respectively, is assembled. In the case of the single-lumen valve-end connector 40, the proximal end of the catheter 54 and the distal end of the septum 75 are bonded into the tubular channel 85 of the snap-in insert 74. The housing 73 is then slid over the sleeve 52 and the distal end of the catheter 54 is inserted through the proximal end of the sleeve 52 and passed completely therethrough. The housing 73 is drawn proximally along the sleeve 52 until the snap-in insert 74 is drawn into the housing 73 past the annular shoulder 77 in the passageway 76 thereof and snapped into position such that the locking shoulder 82 and tapered edge 83 of the fins 81 of the snap-in insert 74 are positioned adjacent the annular shoulder 77 and tapered portion 84 of the channel 76, with the proximal end of the sleeve 52 frictionally held therebetween.

The manifold-end connector 40 is then assembled by first passing the sleeve attachment ring 47 over the distal end 40 of the catheter 54 and over the sleeve 52. Then the magnifying insert 48 is passed over the distal end 63 of the catheter 54 until the distal end 63 extends a predetermined distance beyond the magnifying insert 48. The sleeve 52 is then extended over the magnifying insert 48 and the sleeve attachment ring 47 is pushed onto the cylindrical extension 50 of the magnifying insert 48 to frictionally fit therewith and trap the sleeve 52 therebetween. The remainder of the sleeve 52 extending beyond the distal end of the attachment ring 47 is then folded or rolled back over the attachment ring 47. A sealing ring 58 is then inserted into the annular locking shoulders 56 of the housing 73 and the entire sub-assembly consisting of the magnifying insert 48, the sleeve attachment ring 47, and the portion of the sleeve 52 wrapped around the sleeve attachment ring 47, are then inserted into the housing 42 through the base ring 45 until the annular locking shoulder 57 of the magnifying insert 48 snaps into, and locks behind, the annular locking shoulder 56 of the housing 42 where it presses against the sealing ring 58 in an air-tight manner.

When assembled in this manner, the sleeve 52 is attached to the manifold-end connector 40 such that the catheter 54 can be withdrawn through the connector 40 at least so far as to allow the ring marking 61 thereon to be positioned within the lens 49 of the magnifying insert 48, and to allow the adaptor 43 to protect the distal end 63 of the catheter 54 when the connector 40 is being attached to the manifold 11.

If it is desired to assemble a dual-lumen suction catheter device 41, the valve-end connector 88 thereof (as shown in Figure 11) must first be assembled by passing the attachment ring 104 thereof over the proximal end 98 of the catheter 96 and the proximal end of the sleeve 52 and then inserting the proximal end 98 of the catheter 96 into the second cylindrical opening 94 until a large lumen 97 thereof is securely attached to the tubular extension 95, (being careful, of course, not to inadvertently block the flow channel 100 by the proximal end 98 of the catheter 96). The catheter 96 can then be permanently affixed within the cylindrical opening 94 by any known means such as by solvent bonding or the like. The proximal end of the sleeve 52 is then wrapped around the attachment ring 104 and the attachment ring 104 is affixed by friction fit to the housing 89. Any part of the sleeve 52 extending beyond the proximal side of the attachment ring 104 can then be trimmed off if desired.

The slitted septum 91 is then forced into the cylindrical opening 90 until it engages with the annular shoulder 92 therein, and the one-way valve 103 is inserted into the L-shaped tubular member 101 which itself is then permanently attached to the housing 89 around the flow channel 100 thereof.

The remaining assembly operations of the dual-lumen version of the suction catheter device 41 of the present invention are identical to the assembly of the single-lumen version described above.

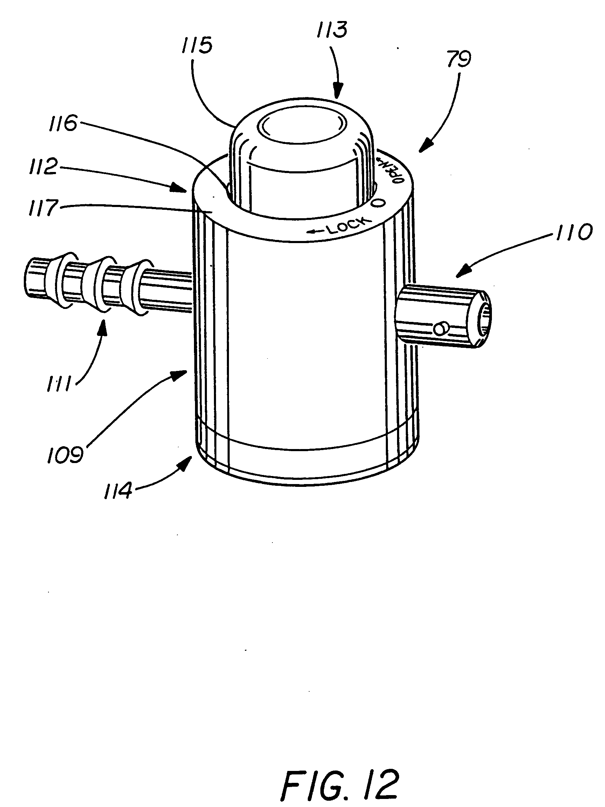

As shown in Figure 12, the valve 79 of the present invention is formed of a valve housing 109 with a suction catheter device connector 110 extending away therefrom in a radial direction and a suction pressure source connector 111 extending away therefrom in a radial direction opposite the connector 110. A lower cap 114 having the same diameter as the valve housing 109, covers the bottom of the valve housing 109. An upper cap 112 is connected to the top of the valve housing 109. A portion of the actuator 113 (constituting the button 115) extends from the interior of the valve housing 109 through the upper cap opening 116 and above the annular surface 117 of the upper cap 112. The positioning of the actuator 113 on the valve 79 is intended to allow for ease of manipulation thereof in a single hand of the user. The valve 79 is sized so as to be easily placeable within a user's palm such that the user's thumb may rest comfortably on the button 115 of the actuator, with the user's fingers curling about the lower cap 114 to support the valve 79 against the internal bias of the actuator 113 when the user presses on the button 115 to open a suction channel through the valve 79.

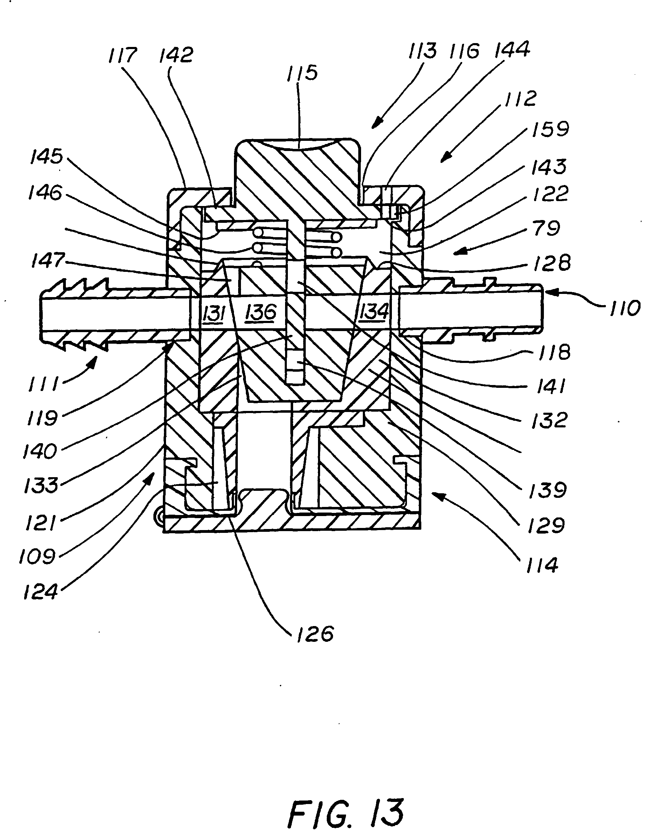

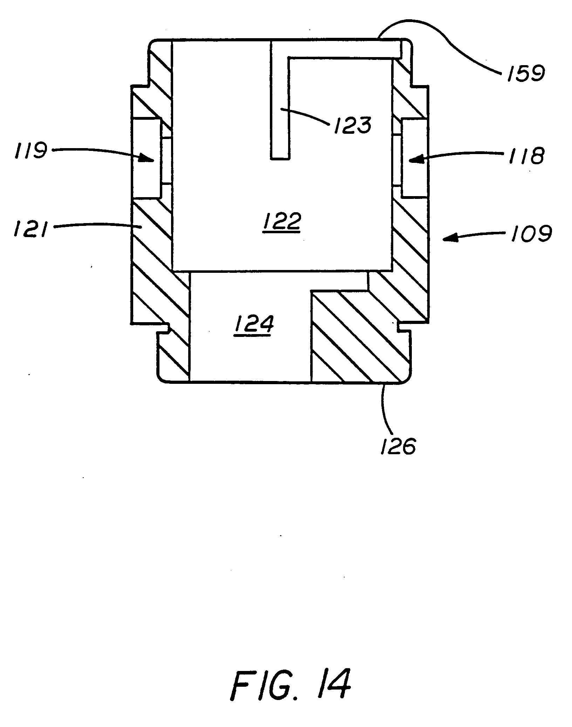

Referring now to Figure 13, the preferred internal structural arrangement of the valve 79 of the present invention will be explained, with the aid of Figures 14-16 which show various views of individual component.

Referring specifically to Figures 13 and 14, the valve housing 109 is formed generally into a hollow cylindrical shape and includes a suction catheter device connector opening 118 and a suction source connector opening 119 which are famed through the aide wall 121 at diametrically opposed positions and which pass into the large cylindrical chamber 122. The openings 118 and 119 each allow attachment of the suction catheter device connector 110 and the suction source connector 111 respectively to the valve housing 109.

The large cylindrical chamber 122 includes a longitudinally oriented groove 123 (best shown in Fig. 14) which aligns with a nub (not shown) on the valve body 125 when the valve body 125 is inserted thereinto in order to ensure their proper relative orientation for use.

The housing 109 also includes a small cylindrical chamber 124 which opens into the large cylindrical chamber 122 and is open through the bottom 126 of the housing 109, which forms part of the ancillary fluid flow channel through the valve 79 as will be explained below.

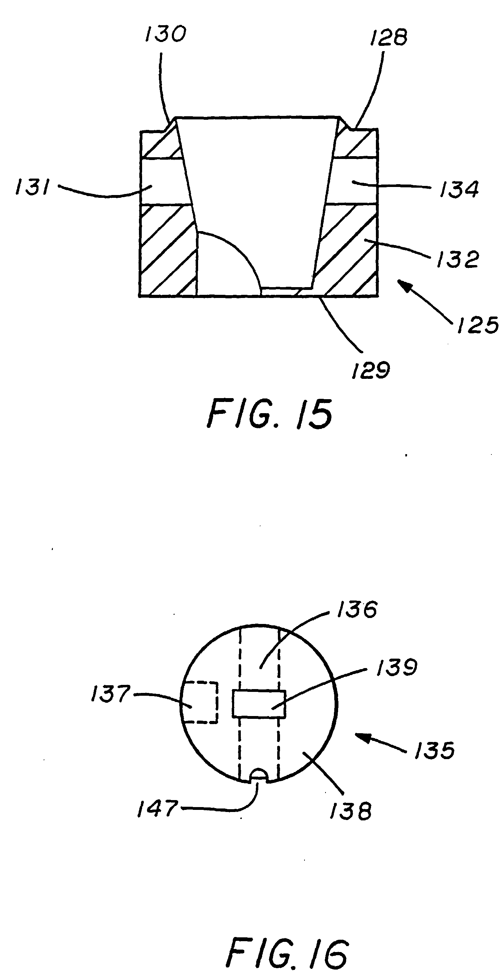

As best seen in Figures 13 and 15, the valve body 125 is a generally cylindrical member having a plurality of openings therethrough. First, a generally conically shaped bore 126 is famed through the top surface 127 and extends nearly to the bottom surface 128 thereof. The conical bore 126 is surrounded at its opening adjacent the top surface 127 by an annularly shaped protrusion or seat 129. A cylindrical fluid flow channel, identified far simplicity of later explanation of operation of the valve 79 as elements 130 and 133, passes through the valve body 125 and completely through the side wall 131 thereof. The channel 130,133 is oriented such that its longitudinal axis perpendicularly intersects with the longitudinal axis of the conical bore 126. A second cylindrical fluid flow channel 132, generally perpendicular to the first fluid flow channel 130,133 passes through the bottom 128 of the valve body 125 into the conical bore 126.

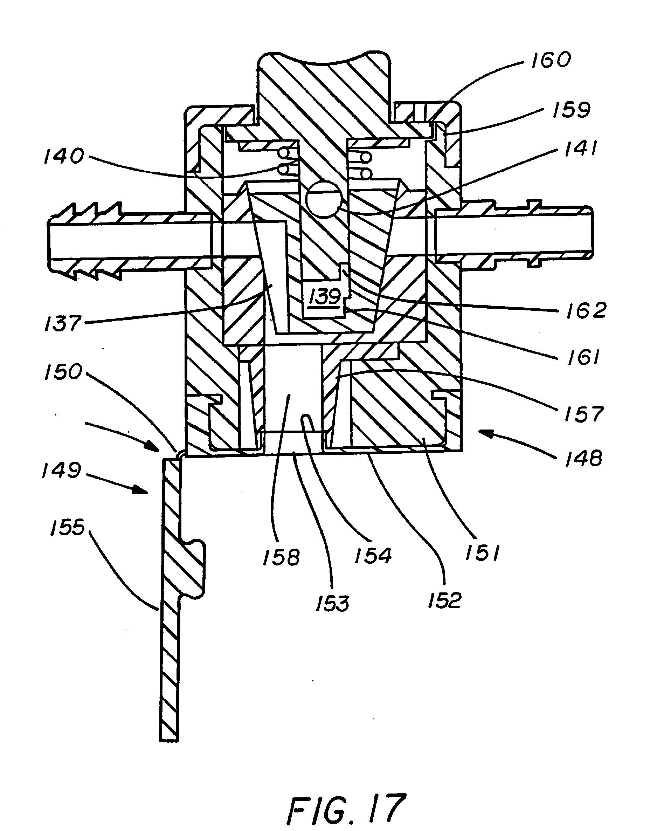

As best seen in Figures 13 and 16, the core 134 rests within the conical bore 126, and is generally conical in shape to match the shape of the conical bore 126. The core 134 forms several channels therethrough which can be positioned for operation of the valve 79 by rotation of the core 134 relative to the body 125 in the manner as will be described below.

A primary fluid flow channel 135 is formed through the core 134 so as to match the diameter of, and be alignable with, the fluid flow channels 130 and 133 in the body 125. An ancillary fluid flow channel 136 (best shown in Figures 16 and 17) attaches fluid flow channel 130 with the second cylindrical fluid flow channel 132. The ancillary fluid flow channel 136 is positioned about the core 134 so as to be oriented approximately one quarter of the way around the circumference of the core from the primary fluid flow channel 135, or in other words (as best seen in Figure 16) the position of the ancillary fluid flow channel 136 is approximately 90° around the surface 137 of the core 125 from the primary fluid flow channel 135. The relative positioning of the primary and ancillary fluid flow channels 135 and 136 respectively, allow positioning of the core 134 in a first position (shown in Figure 13) in which the primary fluid flow channel 135 is oriented for fluid flow between fluid flow channels 130 and 133, and a second position (as shown in Figure 17) in which it is rotated 90° from the first position and in which the ancillary fluid flow channel 136 thereof is in alignment between fluid flow channel 130 and the second cylindrical fluid flow channel 132. The operation of the valve 79 with respect to each position of the core 134 will be explained in detail momentarily.

The core 134 includes a bleed channel 146 which extends from the top surface 137 of the core 134 into the primary fluid channel 135.

The core 134 also includes a rectangularly shaped slot 138 which passes through the top surface 137 of the core and bisects the primary fluid flow channel 135. As can be seen in Figures 13 and 17, the slot 138 accommodates the actuator extension 139 for sliding movement therein between a first position in which the extension 139 blocks flow through the primary fluid flow channel 135, and a second position in which the actuator 113 is forced downwardly to move the actuator opening 140 into alignment with the primary fluid channel 135.

The extension 139 also allows the actuator 113 to effect rotation of the core 134 when the button 115 of the actuator is rotated relative to the valve 79. Rotation of the core 134 between the first or open position as shown in Figure 13, and the lack position shown in Figure 17, is caused by rotation of the button 115 relative to the valve 79 approximately one quarter turn. The valve 79 may include markings such as on the surface 117 of the upper cap 112 (as shown in Figure 12) and/or on the button 115, to indicate the position of the core 134 for proper operation of the valve.

As shown in Figure 13, the actuator 113 includes a shoulder 141 which is sized to fit within the large cylindrical chamber 122 of the housing 109 to be held in place there within by the upper cap 112. The shoulder 141 forms a slot 142 therethrough which is located adjacent the opening 143 in the upper cap 112 when the actuator 113 is in its first or open position. The shoulder 141 also includes a tab 159 (shown only in Fig. 17) which is positioned around the circumference of the shoulder 141 approximately 90° away from the slot 142. The tabs 159 rides in the slot 158 (best shown in Fig. 14) formed around one quarter (90°) of the internal circumference of the large cylindrical chamber 122 of the housing 109 and joins with the longitudinally oriented groove 123. As is evident, the tab 159 allows the actuator 113 to rotate only a quarter turn, since it is inhibited from further rotation by the ends of the slot 158. In Figure 13 for example, the tab 159 is rotated to the end of slot 158 which is adjacent the longitudinal groove 123. In this position, the slot 142 through the shoulder 141 of the actuator button 115 is positioned adjacent the opening 143 in the upper cap 112. In Figure 17, the tab 159 is rotated one quarter of a turn to the opposite end of slot 158 and is positioned directly adjacent the opening 143 in the upper cap 112. It should be noted that the width of the tab 159 is less than the width of the groove 123 so that the tab 159 may pass downwardly therethrough whenever it is aligned therewith and the button 115 of the actuator 113 is pushed down. As is readily evident therefor, only single rotational position of the actuator 113 allows downward movement thereof, this being defined as the "open" position where the tab 159 is aligned with the groove 123.

Referring again to Figure 13, a flexible, soft elastomeric pad 144 of generally circular shape is affixed to the actuator 113 below the shoulder 141 and is of a slightly larger diameter than the diameter of the seat 129 protruding from the surface 127 of the body 125. A compression spring 145 is positioned between the top surface 137 of the core 134 and the shoulder 141 and operates to hold the actuator 113 in its uppermost position where the actuator shoulder 141 abuts the upper cap 112.

The fluid flow passage formed by the suction source connector 111, the fluid flow channels 130, 135, 133, and the suction catheter device connector 110, forms essentially an elongate linear channel of uniform diameter passing entirely through the valve 79. When the actuator opening 140 is moved downwardly to be positioned within the primary fluid flow channel 135, it is readily apparent that a single linear fluid flow channel of uniform diameter through the entire valve 79 is formed which does not cause any obstruction or blockage of fluid passing through the valve 79. In this manner, mucal material, including clotted material referred to generally as mucus plugs, encounters no obstruction as it is drawn through the valve 79, and therefore is not likely to cause blockage of the valve during use.

As shown in Figures 13 and 17, the bottom of the valve housing 109 is covered with a lover cap or "flip cap" 114. The lower cap 114 is formed of a generally cylindrical shape having a diameter equal to the diameter of the valve housing 109 and includes a fixed member 147 which is hingeably attached to a cover member 148 by means of hinge 149 which may be of the "living hinge" type and formed of polymeric material. The fixed member 147 is preferably attached to the annular base 150 of the valve housing by a snap fit or an ultrasonic weld, however any well known attachment means may be used. The fixed member 147 includes a circular plate 151 which has an opening 152 formed centrally therein which is surrounded by an inwardly projecting boss 153. The cover member 148 also includes a circular plate 154, on the interior surface of which a plug 155 is formed and sized so as to fit snugly within the fixed member opening 152 to from a fluid tight seal whenever the cover member 148 is closed over the fixed member 147.

A bushing 156 is located within cylindrical chamber 124 of the valve housing 109 between the body 125 and the circular plate 151. The bushing 156 forms a fluid flow channel 157 therethrough which is shaped on one end thereof to connect with boss 153 on the circular plate 151 and on the other end to align with the second cylindrical fluid flow channel 132 of the body 125 in fluid tight relationship, thus allowing fluid flow connection of the bushing fluid flow channel 157 with the suction source through the ancillary fluid flow passage 136.

It should also be noted that all internal components of the valve 79, including the actuator 113, core 134, body 125, and bushing 156 are designed such that assembly thereof into the valve housing 109 is substantially simplified. In each instance, the particular element to be assembled into the valve housing 109 has been designed to the extent possible to allow only the element to fit within the valve housing 109 only when it is properly positioned for assembly. Specifically, the bushing 156 is formed asymmetrically to allow only one possible positioning thereof within the small cylindrical chamber 124. The body 125 includes a nub (not shown) which must be aligned with groove 123 in order for the body 125 to be insertable within the large cylindrical chamber 122. The core 125 includes a step 66 in the bottom of the slot 138 thereof which accommodates the actuator extension 139 forces proper alignment of the actuator extension 139 therein by allowing proper operation only when shoulder 67 of the extension 139 is oriented in alignment therewith. The actuator 113/core 125 sub-assembly can only be positioned within the valve housing 109 such that the tab 159 of the shoulder 141 of the actuator button 115 is positioned within the slot 158 of the housing 109. Although various methods and means for ensuring proper assembly of the valve 79 of the present invention have been shown, it should be understood that other means and methods known in the art could also be employed without departing from the spirit and scope of the present invention.

### OPERATION OF THE PREFERRED EMBODIMENTS

Operation of the respiratory support system 10 is preferably as follows. First, the ventilator manifold 11 is attached to the tracheal tube which has previously been inserted into the patient's trachea, and the ventilator circuit of the respiratory support system is attached to the manifold 11 in a well-known manner. The manifold-end connector 40 of the suction catheter device 41 is then inserted into the access port 15 of the manifold 11 and rotated to its above-described locking position therewith. The suction control valve 79 is then inserted into the valve-end connector 72 and then attached to a source of suction pressure in a well-known manner.

When in its first or open position as shown in Figure 13, the actuator 113 of the control valve 79 allows a bleed of auctioned atmospheric air to pass into the valve 79 through the cap bleed opening 143 and move past the actuator shoulder slot 142 into the large cylindrical chamber 122 of the valve housing 109 where it is then drawn through the core bleed channel 146 into the primary fluid flow channel 135 of the core 134 and from there through channel 130 and into the suction source connector 111 where it can be drawn out of the valve 79 into the suction pressure source. Movement of atmospheric air through the valve 79 to the suction source when the actuator 113 is in the open position generates an auditory signal, being a very recognizable "hissing" sound, which is indicative of the operation of the suction pressure source and the presence of suction pressure within the valve 79.

When it is desired to suction the patient's trachea or lungs, the catheter 54 is advanced through the manifold-end connector 40, the manifold 11, and the tracheal tube into the patient's trachea and lungs any desired distance (which can be monitored by the medical worker performing the procedure by viewing the number markings 62 which appear through the lens 49 of the connector 40). Aspiration of the patient's trachea and lungs is then performed by the user forcing the actuator button 115 downwardly into the valve housing 109 against the bias of the compression spring 145. This linear translational movement of the actuator 113 relative to the valve housing 109 causes the actuator extension 139 to move downwardly within the core slot 142. This causes the actuator opening 140 to move into alignment with the primary fluid flow channel 135 of the core 134. No resistance of downward movement of the actuator 113 is caused by the tab 159, since it is aligned with groove 123 and can therefore pass downwardly therein.

As can be seen, although the actuator extension 139 blocks the primary fluid flow channel 135 whenever the actuator 113 is in its fully upwardly extended or "released" position, it gradually moves out of blocking position as the actuator opening 140 is moved into alignment position with the primary fluid flow channel 135 as the actuator button 115 is depressed. As is also readily evident, the amount of suction pressure allowed through the primary fluid flow channel 135 can be regulated from a "no flow" level when the button 115 is released, to gradually increasing flow levels as the actuator opening 140 is moved into alignment with the primary fluid flow channel 135 as the button 115 is depressed toward the body 125.

As can be seen, complete depression of the button 115 occurs when the pad 144 on the actuator shoulder 141 contacts and seals against the seat 129 of the body 125. In the completely depressed position, the actuator opening 140 is completely aligned with the primary fluid flow channel 135 and presents no fluid flow obstacle therethrough.

It should be noted that when the actuator 113 is completely depressed until the pad 144 seals against seat 129 causing complete alignment of the actuator opening 140 with the primary fluid flow channel 135, fluid flow caused by the suction pressure source is allowed to pass directly through the valve 79 in a completely open and linear flow path, having no element of the valve 79 obstructing the passage of flow therethrough. This is especially useful in the preferred intended use of the valve 79 of the present invention of suctioning fluids from a patient's trachea and lungs, since it affords the clearest possible passageway through the valve 79 for fluids normally suctioned from the patient. Even clotted mucal material can pass easily through the valve 79 without the risk of clogging the fluid flow passages therethrough since there are no obstructing valve elements.

Complete depression of the button 115 causes the pad 144 to seal against seat 129 and block the flow of atmospheric air through bleed channel 146. Thus, whenever the actuator button 115 is depressed, bleeding of atmospheric air into the primary fluid flow channel 135 is prevented. This causes the "hissing" of the valve 79 to stop, which provides the user with another audio indication of the proper operation of the valve 79. The user immediately recognizes the arresting of the "hissing" sound upon depression of the actuator button 115, which signals the user that the suction pressure has been diverted into the suction catheter device 41. In this way, the presence or absence of the "hissing" sound provided by the valve 79 of the present invention assists the user in confining proper operation of the valve 79.

When the actuator button 115 is released after suctioning through the suction catheter device 41 is completed, the actuator opening 140 moves upwardly, due to the bias of the compression spring 145, to again allow atmospheric air to pass through the valve 79, and generate the "hissing" auditory signal. Upward movement of the actuator 113 is arrested by the abutment of the actuator shoulder 141 against the upper cap 112.

The catheter 54 is then withdrawn until the medical worker can view the ring marking 61 through the lens 49. The medical worker may then clean the distal end of the catheter 54 by injecting fluid through the access port side opening 22 and the injection fluid opening 69 of the adapter 43, and subsequently suctioning the fluid through the catheter 54.

Alternatively, a medical worker may inject lavage fluid through the access port side opening 22 into the adapter 43 and allow it to pass into the manifold 11 and down the patient's trachea and lungs, and thereafter insert the catheter 54 into the patient's trachea and lungs to aspirate the patient to remove the lavage fluid.

At times it is convenient, and even important from a safety consideration for a patient, to ensure that depression of the actuator button 115 cannot allow suction pressure through the suction catheter device 41. If it is desired to prevent suctioning through the suction catheter device 41, the user may rotate the actuator 113, by rotating actuator button 115, approximately one quarter turn to the locked position.

As best shown in Figure 17, one quarter rotation of the actuator 113 causes the core 134 to also rotate within the body 125 approximately one quarter turn. In this position, the ancillary fluid flow channel 136 is positioned in direct alignment with the fluid flow channel 130 of the body 125, and any incidental depression of the actuator button 115 when in this locked position will fail to allow fluid flow Through the primary fluid flow passage 135, since it has been moved out of alignment with fluid flow channel 130. Therefore, no suction pressure can be applied to the suction catheter device 41.

Further, whenever the actuator 113 has been rotated to the locking position, the bleed channel 146 of the core 134 is also rotated out of alignment with the fluid flow channel 130. Therefore, bleed of atmospheric air into the primary fluid flow passage 135 is prevented, and the user is aware of such by the arresting of the "hissing" auditory signal.

This feature of the present invention allows a user to lock the actuator 113 against accidental suctioning through the suction device 41 (such as may occur if the valve 79 and suction catheter device 41 are left unattached while attached to a respiratory support system on a patient). Although a patient may inadvertently depress the actuator 113, for example, by accidentally rolling over on top of the valve 79, suctioning of fluid through the suction catheter device 41 cannot occur since the actuator 113 is in the locked position, with tab 159 thereof rotated out of position with groove 123 of the valve housing 109, and the core 134 rotated to block fluid flow through channel 133.

Further, medical personnel or other users of the valve 79 will be provided with an auditory signal (absence of hissing) whenever the valve 79 is locked against actuation, and a different auditory signal (the presence of hissing) whenever the valve 79 is unlocked or opened. This can be extremely convenient and add an additional safety factor to the use of the valve 79 in that it is not necessary for the user to see directly whether or not the valve 79 is locked against actuation, because an auditory hissing signal is generated whenever the valve 79 is open, which signals the user that the valve 79 must either be attended to, or locked, in order to avoid possible injury to the patient.

The valve 79 of the present invention may also operate as a connector for an ancillary suctioning device such as a Yankauer suction wand (not shown) if desired. As shown in Figure 7, when it is desired to attach an ancillary device to the valve 79 of the present invention, the user merely rotates the cover member 148 of the lower cap 114 to an open position. The end connector of the Yankauer suction wand or other ancillary device is then inserted through the fixed member circular plate opening 152 and into the bushing fluid flow channel 157 to generate a friction fit therewith to hold the Yankauer in connection with the valve 79. As can be seen, attachment of a Yankauer in this manner provides immediate connection thereof with the suction pressure source attached to the valve 79 through the bushing fluid flow channel 157 and the valve housing fluid flow channel 130 whenever the actuator 113 is in the locked position.

Attachment of an ancillary device to the valve 79 without requiring detachment of the suction catheter device 41 therefrom can be very important in any procedures involving suctioning of fluids from a patient attached to a respiratory support system. Since serious detriment to the patient can occur whenever it is necessary to breach the integrity of the respiratory support system, the avoidance of disassembly of any equipment thereof, or detachment of the suction source, becomes a positive procedural improvement.

As can be seen with the present invention, the ability to attach a Yankauer suction wand to the valve 79 to allow suctioning of the patient's oral cavity without the necessity of disassembling any part of the system in place for primary auctioning of the patient's trachea and lungs is an important improvement over the prior art.

When the Yankauer suction wand is no longer needed, it can be detached from the valve 79 and the cover member 148 can again be closed to block the bushing fluid flow channel 147 and seal it against fluid flow therethrough.

It should be noted that suctioning through the ancillary port connection of the valve 79 can only be accomplished when the actuator 113 is in the locked position, with the ancillary fluid flow channel 136 oriented for fluid flow with the fluid flow channel 130.

When it becomes necessary to remove the suction catheter device 41 from the manifold 11, the manifold-end connector 40 is merely detached from the access port 15 and withdrawn therefrom. Alternatively, if it becomes necessary to replace the suction control valve 79, it can be disconnected from the valve-end connector 72 and replaced. In either instance, no loss of PEEP from the manifold 11 occurs due to the normally closed manifold valve 16 of the manifold 11 and/or the slitted septum 75 of the valve-end connector 72.

Use of the alternative embodiment of the suction catheter device 41 which includes the dual-lumen catheter 96 and the dual-lumen valve-end connector 88 is similar to that described above. However it includes the added feature of allowing lavage fluid to be injected into the patient's trachea and/or lungs through the irrigation lumen 99 of the dual-lumen catheter 96. This is done by placing a flexible fluid vial 87 in fluid connection with the luer connector opening 102 of the valve-end connector 88 and injecting fluid through the L-shaped tubular member 101 and the flow channel 100 into the irrigation lumen 99. This method of injecting irrigation fluid for lavage has the added feature of injecting the fluid directly from the distal end of the catheter 96 to allow more directed and forceful fluid flow into the patient's trachea and lungs.

If desired, "time-in-use" markings nay be placed on the manifold 11, the suction catheter device 41, and/or the suction control valve 79 to provide the medical worker with an indication of the amount of time the particular component has been part of the respiratory support system. Further, if desired, the "time-in-use" markings may indicate a recommended time period for use, and further if desired, may include means for indicating of the amount of time which has passed since the component has been assembled within the respiratory support system. An example of such means is a color change indicator which can be actuated when the component is attached as part of the respiratory support system 10 and will change colors at a predetermined time period to signal the medical worker when the component is due to be changed out of the respiratory support system 10. Other indicators may be used, such as marking areas, tags, etc. and remain within the spirit and scope of the present invention.

It should be understood from the foregoing that, while particular embodiments of the invention have been illustrated and described, various modifications can be made thereto without departing from the spirit and scope of the invention. Therefore, it is not intended that the invention be limited by the specification; instead, the scope of the present invention is intended to be limited only by the appended claims.

## Claims

1. A respiratory support system (10) comprising:
manifold means (11) having ports (12,13) adapted to be connected for fluid flow attachment between a patient and a ventilator circuit, and
a suction catheter device (41) comprising tubular connector means (40), a sleeve (52) attached to said connector means (40) and aligned with the through passage thereof, and a catheter (54) within said sleeve (52) and slidable through said connector means (40), said manifold means (11) further including an access port (15) for allowing attachment of said connector means (40) to said manifold means (11), said access port (15) being normally closed against fluid flow therethrough by a valve, in use attachment of said connector means (40) to said access port (15) operating to open said valve to allow fluid flow access between said manifold means (11) and said suction catheter (41), characterized in that said valve comprises a resilient disc (16) having a normally closed slit (20) therein, whereby during operation said connector means (40) opens said slit (20) and sealingly engages said disc (16) and in that said connector means (40) comprises a locking mechanism (44) to ensure secure attachment of the connector means (40) to said access port (15) to prevent inadvertent withdrawal thereof during use.

2. A system (10) according to claim 1 wherein said access port (15) further includes an injection fluid inlet (22) therein, and said connector means (40) has an injection fluid opening (69) to allow fluid flow access between said injection fluid inlet (22) and said manifold means (11) via said disc valve (16).

3. A system (10) according to claim 2 wherein said connector means (40) is tubular and said injection fluid opening (69) comprises an aperture in the wall thereof, said connector means (40) having alignment means (67,71) to ensure alignment of said injection fluid inlet (22) and said opening (69).

4. A system (10) according to any preceding claim wherein said locking mechanism (44) comprises a detent and stop type locking arrangement.

5. A system (10) according to claim 4, wherein said connector means (40) includes a window (55) for viewing said catheter (54) therethrough.

6. A system (10) according to claim 5 wherein said window (55) includes a lens (49) .

7. A system (10) according to any of claims 4-6 wherein said catheter includes a series of markings (61,62) along the length thereof.

8. A system (10) according to any preceding claim wherein a proximal end connector means (72) is adapted for fixed attachment to the proximal end of said catheter (54), for connecting said suction catheter device (41) to a suction control means (79); means (75) being provided for preventing fluid flow through said catheter (54) when said proximal end connector means (72) is not attached to the suction control means (79), and for allowing fluid flow through said catheter (54) when said proximal end connector means (72) is attached to the suction control means (79).

9. A system (10) according to claim 8 wherein said proximal end connector means (72) has an air flow passage (81) therethrough, said passage (81) allowing passage of air through said proximal end connector means (72) independent of said means (75) for preventing fluid flow through said catheter (54).

10. A system (10) according to claim 8 or claim 9 wherein said means (75) for preventing fluid flow comprises a slit septum.

11. A system (10) according to any of claims 8-10 wherein said catheter (54) has a second lumen (99) and said proximal end connector means (88) includes means (101) for injecting fluid therethrough into said second lumen (99).

12. A system (10) according to any preceding claim and further including a suction control valve (79) comprising a valve housing (109) having a suction source access port (119), a primary suction device access port (118) , and a fluid flow passage for allowing fluid flow between said suction source access port (119) and said primary suction device access port (118),
movable actuator means (113) positioned at least partially within said valve housing (109) for movement relative thereto between at least a first position in which said primary suction device access port (119) is closed against fluid flow therethrough, and a second position in which said primary suction device access port (119) is open to fluid flow therethrough, and
auditory signal means (143, 144, 147) for indicating the location of said actuator means (113) between said first and said second positions.

13. A system (10) according to claim 12 wherein said suction control valve (79) further includes an ancillary suction device access port (158) in fluid flow connection with said fluid flow passage.

## Patentansprüche

1. Ein Atmungsunterstützungssystem (10) mit:
Verteilermitteln (11), welche Ports (12,13) aufweisen, um mit einer Fluidflußausrüstung zwischen einem Patienten und einem Ventilationskreislauf verbunden zu werden, und
einer Saugkathetervorrichtung (41) welche umfaßt: röhrenförmige Verbindungsmittel (40), eine Manschette (52), welche an den Verbindungsmitteln (40) angebracht ist und mit dessen Durchgangspassage ausgerichtet ist und einem Katheter (54) innerhalb der Manschette (52) und gleitbar durch diese Verbindungsmittel (40), wobei die Verteilermittel (11) weiter einschließen: einen Zugangsport (15), um den Anschluß der Verbindungsmittel (40) an die Verteilermittel (11) zu erlauben, wobei der Zugangsport (15) normalerweise mittels eines Ventils gegen Fluidfluß hindurch geschlossen ist, bei Gebrauch, der Anschluß der Verbindungsmittel (40) an den Zugangsport (15) betrieben wird, um das Ventil zu öffnen, um einen Fluidflußzugang zwischen dem Verbindungsmittel (11) und dem Saugkatheter (41) zu erlauben, dadurch gekennzeichnet, daß das Ventil eine elastische Scheibe (16) aufweist, welche normalerweise einen geschlossenen Schlitz (20) darin aufweist, wodurch sich während des Betriebs des Verbindungsmittels (40) der Schlitz (20) öffnet und siegelnd in die Scheibe (16) eingreift und daß das Verbindungsmittel (40) einen Verschlußmechanismus (44) aufweist, um ein sicheres Anbringen des Verbindungsmittels (40) an dem Zugangsport (15) zu gewährleisten, um ein unabsichtliches Abziehen während des Gebrauchs zu verhindern.

2. Ein System (10) nach Anspruch 1, wobei der Zugangsport (15) ferner einen Injektionsfluideinlaß (22) darin einschließt und das Verbindungsmittel (40) eine Injektionsfluidöffnung (69) aufweist, um einen Fluidflußzugang zwischen dem Injektionsfluideinlaß (22) und dem Verteilermittel (11) über das Scheibenventil (16) zu erlauben.

3. Ein System (10) nach Anspruch 2, wobei das Verbindungsmittel (40) röhrenförmig ist und die Injektionsfluidöffnung (69) eine Öffnung in dessen Wand umfaßt, wobei das Verbindungsmittel (40) Ausrichtungsmittel (67,71) aufweist, um die Ausrichtung des Injektionsfluideinlasses (22) und der Öffnung (69) sicherzustellen.

4. Ein System (10) nach irgendeinem der vorhergehenden Ansprüche, worin der Verschlußmechanismus (44) eine Verschlußeinrichtung vom Arretier- und Stopptyp umfaßt.

5. Ein System (10) nach Anspruch 4, wobei das Verbindungsmittel (40) ein Fenster (55) zum Beobachten des Katheters (54) hierdurch einschließt.

6. Ein System (10) nach Anspruch 5, worin das Fenster (55) eine Linse (49) einschließt.

7. Ein System (10) nach irgendeinem der Ansprüche 4-6, worin der Katheter eine Reihe von Markierungen (61,62) entlang seiner Länge einschließt.

8. Ein System (10) nach irgendeinem der vorhergehenden Ansprüche, worin ein Verbindungsmittel (72) am proximalen Ende ausgelegt ist zur fixierenden Anordnung des proximalen Endes des Katheters (54) zum Verbinden der Saugkathetervorrichtung (41) mit einem Saugkontrollmittel (79); wobei Mittel (75) vorgesehen sind zum Verhindern eines Fluidflusses durch den Katheter (54) wenn das Verbindungsmittel (73) am proximalen Ende nicht an das Saugkontrollmittel (79) angeschlossen ist und zum Erlauben eines Fluidflusses durch den Katheter (54), wenn das Verbindungsmittel (72) am proximalen Ende an das Saugkontrollmittel (79) angeschlossen ist.

9. Ein System (10) nach Anspruch 8, worin das Mittel am proximalen Ende (72) eine Luftflußpassage (81) hierdurch aufweist, wobei die Passage (81) den Durchtritt von Luft durch das Verbindungsmittel (72) am proximalen Ende erlaubt, unabhängig von dem Mittel (75) zum Verhindern eines Fluidflusses durch den Katheter (54).

10. Ein System (10) nach Anspruch 8 oder Anspruch 9, worin das Mittel (75) zum Verhindern eines Fluidflusses eine geschlitzte Scheidewand umfaßt.

11. Ein System (10) nach irgendeinem der Ansprüche 8-10, worin der Katheter (54) ein zweites Lumen (99) aufweist und das Verbindungsmittel (88) am proximalen Ende ein Mittel (101) zum Injizieren eines Fluides hierdurch in das zweite Lumen (99) einschließt.

12. Ein System (10) nach irgendeinem der vorgehenden Ansprüche und weiter einschließend ein Saugkontrollventil (79), welches umfaßt: ein Ventilgehäuse (109) mit einem Saugquellenzugangsport (119), einen primären Saugeinrichtungszugangsport (118) und eine Fluidflußpassage, um einen Fluidfluß zwischen dem Saugquellenzugangport (119) und dem primären Saugeinrichtungszugangport (118) zu erlauben,
bewegbare Betätigungsmittel (113), angeordnet wenigstens teilweise innerhalb des Ventilgehäuses (109) zum Bewegen relativ hierzu zwischen wenigstens einer ersten Position in welchem der primäre Saugeinrichtungszugangsport (119) geschlossen ist für einen Fluidfluß hierdurch, und einer zweiten Position, in welcher der primäre Saugeinrichtungszugangsport (119) offen für einen Fluidfluß hierdurch ist, und
hörbare Signalmittel (143,144,147) zum Anzeigen der Stellung der Betätigungsmittel (113) zwischen den ersten und zweiten Positionen.

13. Ein System (10) nach Anspruch 12, worin das Saugkontrollventil (79) ferner einschließt einen ergänzenden Saugeinrichtungszugangsport (158) in Fluidflußverbindung mit der Fluidflußpassage.

## Revendications

1. Système d'assistance respiratoire (10) comprenant :
un distributeur (manifold) (11) comportant des ports (12, 13) destinés à être connectés pour permettre le raccordement d'écoulements de fluide entre un patient et un circuit de respirateur artificiel, et
un dispositif de cathéter d'aspiration (41) comprenant un connecteur tubulaire (40), un manchon (52) relié au connecteur (40) et aligné avec son passage traversant, ainsi qu'un cathéter (54) placé à l'intérieur du manchon (52) et pouvant glisser à travers le connecteur (40),
le distributeur (11) comprenant en outre un port d'accès (15) pour permettre le raccordement du connecteur (40) au distributeur (11), ce port d'accès (15) étant normalement fermé par une soupape pour empêcher le fluide de s'écouler à travers celui-ci, et le raccordement entre le connecteur (40) et le port d'accès (15) servant, en cours d'utilisation, à ouvrir la soupape pour permettre l'accès de l'écoulement de fluide entre le distributeur (11) et le cathéter d'aspiration (41),
caractérisé en ce que
• la soupape comprend un disque élastique (16) dans lequel est formée une fente normalement fermée (20) de façon que, pendant le fonctionnement, le connecteur (40) ouvre la fente (20) et s'engage de manière étanche contre le disque (16), et
• le connecteur (40) comprend un mécanisme de verrouillage (44) pour assurer un raccordement de sécurité entre le connecteur (40) et le port d'accès (15) pour éviter une extraction intempestive de celui-ci en cours d'utilisation.

2. Système (10), selon la revendication 1,
dans lequel
• le port d'accès (15) comprend en outre une entrée de fluide d'injection (22) dans celui-ci, et
• le connecteur (40) comporte une ouverture de fluide d'injection (69) pour permettre l'accès de l'écoulement de fluide entre l'entrée de fluide d'injection (22) et le distributeur (11) par l'intermédiaire de la soupape à disque (16).

3. Système (10),selon la revendication 2
dans lequel
le connecteur (40) est tubulaire et l'ouverture de fluide d'injection (69) comprend une ouverture dans sa paroi, le connecteur (40) comportant des moyens d'alignement (67, 71) pour assurer l'alignement de l'entrée de fluide d'injection (22) avec l'ouverture (69).

4. Système (10),selon l'une quelconque des revendications précédentes,
dans lequel
un mécanisme de verrouillage (44) comprend un dispositif de verrouillage de type à détente et butée.

5. Système (10),selon la revendication 4,
dans lequel
le connecteur (40) comprend une fenêtre (55) pour qu'on puisse voir le cathéter (54) à travers celle-ci.

6. Système (10),selon la revendication 5,
dans lequel
la fenêtre (55) comprend une lentille (49).

7. Système (10),selon l'une quelconque des revendications 4 à 6,
dans lequel
le cathéter comprend une série de marquages (61, 62) suivant sa longueur.

8. Système (10),selon l'une quelconque des revendications précédentes,
dans lequel
• un connecteur d'extrémité proximal (72) est destiné à se raccorder de façon fixe à l'extrémité proximale du cathéter (54) pour relier le dispositif de cathéter d'aspiration (41) à un moyen de commande d'aspiration (79),
• un moyen (75) étant prévu pour empêcher l'écoulement de fluide à travers le cathéter (54) lorsque le connecteur d'extrémité proximale (72) n'est pas relié au moyen de commande d'aspiration (79), et pour permettre l'écoulement de fluide à travers le cathéter (54) lorsque le connecteur d'extrémité proximale (72) est relié au moyen de commande d'aspiration (79).

9. Système (10),selon la revendication 8,
dans lequel
le connecteur d'extrémité proximal (72) comporte un passage d'écoulement d'air (81) à travers celui-ci, ce passage (81) permettant le passage d'air à travers le connecteur d'extrémité proximal (72) indépendamment du moyen (75) pour empêcher l'écoulement de fluide à travers le cathéter (54).

10. Système (10),selon la revendication 8 ou la revendication 9,
dans lequel
le moyen (75) pour empêcher l'écoulement de fluide comprend un septum fendu.

11. Système (10),selon l'une quelconque des revendications 8 à 10,
dans lequel
le cathéter (54) comporte une seconde lumière (99) et le connecteur d'extrémité proximal (88) comprend un moyen (101) pour injecter du fluide à travers celui-ci dans la seconde lumière (99).

12. Système (10),selon l'une quelconque des revendications précédentes,
comprenant en outre
• une soupape de commande d'aspiration (79) comprenant un boîtier de soupape (109) comportant un port d'accès (119) d'une source d'aspiration, un port d'accès (118) d'un dispositif d'aspiration primaire, et un passage d'écoulement de fluide pour permettre l'écoulement de fluide entre le port d'accès (119) de la source d'aspiration et le port d'accès (118) du dispositif d'aspiration primaire,
• un actionneur mobile (113) positionné au moins partiellement à l'intérieur du boîtier de soupape (109), pour se déplacer par rapport à celui-ci entre au moins une première position dans laquelle le port d'accès (119) du dispositif d'aspiration primaire est fermé pour empêcher l'écoulement de fluide à travers celui-ci, et une seconde position dans laquelle le port d'accès (119) du dispositif d'aspiration primaire est ouvert à l'écoulement de fluide à travers celui-ci, et
• des moyens de signaux auditifs (143, 144, 147) pour indiquer la position de l'actionneur (113) entre la première position et la seconde position.

13. Système (10),selon la revendication 12,
dans lequel
la soupape de commande d'aspiration (79) comprend en outre un port d'accès (158) d'un dispositif d'aspiration auxiliaire, en communication d'écoulement de fluide avec le passage d'écoulement de fluide.
